# EUROPÄISCHE PATENTANMELDUNG

(11) **EP 2 452 613 A1**
(43) Veröffentlichungstag der Anmeldung: **16.05.2012**
(21) Anmeldenummer: 11188397.1
(22) Anmeldetag: 09.11.2011
(51) Int. Cl.: A61B 1/24, A61B 5/00, A61B 1/00

(54) **Medizinisches, insbesondere zahnmedizinisches Diagnosegerät mit Mitteln zur Bilderfassung**

(30) Priorität: 11.11.2010 DE 102010043794
(71) Anmelder: Kaltenbach & Voigt GmbH, 88400 Biberach (DE)
(72) Erfinder: Hackel, André, 88400 Biberach (DE)
(74) Vertreter: Thun, Clemens

(57) **Zusammenfassung**

Bei einem medizinischen, insbesondere zahnmedizinischen Diagnosegerät (1) mit einem länglichen Handstück (2) mit Mitteln zur Bilderfassung (27) sowie einem optischen System mit zumindest einem optischen Element (25) zur Umlenkung eines seitlich von dem Handstück (2) einfallenden Lichtstrahls auf die Mittel zur Bilderfassung (27) ist der Umlenkwinkel (α) des zumindest einen optischen Elements (25) kleiner als 90°.

## Beschreibung

Die vorliegende Erfindung betrifft ein medizinisches, insbesondere ein zahnmedizinisches Diagnosegerät gemäß dem Oberbegriff des Anspruchs 1, welches ein längliches Handstück mit Mitteln zur Bilderfassung aufweist, wobei ferner ein optisches System mit zumindest einem optischen Element zur Umlenkung eines seitlich von dem Handstück einfallenden Lichtstrahls auf die Mittel zur Bilderfassung vorgesehen ist. Insbesondere betrifft die vorliegende Erfindung ein Diagnosegerät zum Transilluminieren von Zähnen.

In der Medizin, insbesondere der Zahnmedizin werden vermehrt Diagnosesysteme eingesetzt, welche auf optischen Prinzipien beruhen. Grund hierfür ist, dass derartige Geräte üblicherweise berührungslos, also insbesondere schmerzfrei die Erstellung einer Diagnose ermöglichen und darüber hinaus auch oftmals optische Bilder zur Verfügung stellen, anhand welcher eventuell erforderliche therapeutische Maßnahmen dem Patienten anschaulich und damit besser vermittelt werden können. Beispielsweise kommen in der Zahnmedizin sogenannte Intraoralkameras zum Einsatz, welche ein Handstück beinhalten, dessen vorderer Endbereich in den Mund eines Patienten eingeführt wird. In diesem Endbereich befindet sich in der Regel ein Lichteintritts- bzw. Sichtfenster für die Kameraoptik, von dem ausgehend das Bild des zu untersuchenden Objekts einer Erfassungseinrichtung, beispielsweise einem CCD-Chip übermittelt wird.

Eine derartige Intraoralkamera kann ferner zu einem System zum Transilluminieren von Zähnen erweitert werden, wie es u. a. aus der DE 10 2006 041 020 A1 der Anmelderin bekannt ist. Hierbei wird der zu untersuchende Zahn mit Licht innerhalb eines bestimmten Wellenlängenbereichs bestrahlt, wobei dann ein optisches Bild des durch die Untersuchungsstrahlung illuminierten Zahns erfasst und bewertet wird. Da kariöse Stellen im Zahn das Licht anders streuen als gesundes Zahngewebe, können derartige Stellen bei Beobachtung des Zahns mit Hilfe einer Kamera identifiziert werden, wobei bei entsprechender Ausgestaltung des Systems sogar eine zuverlässigere Kariesdiagnose möglich ist als dies bei einer klassischen Röntgenuntersuchung der Fall ist.

Insbesondere dann, wenn mit Hilfe eines derartigen Transilluminations-Systems molare Zahnflächen beobachtet werden sollen, besteht eine eingeschränkte Bewegungsfreiheit des Geräts aufgrund der Gefahr der Kollision mit dem vorderen Schneidezahnbereich. Die bislang bekannten Geräte weisen üblicherweise zur Bildübertragung ein optisches Element auf, welches eine rechtwinklige Strahlumlenkung bewirkt und das Licht auf die Erfassungseinrichtung, also bspw. den CCD-Chip übermittelt. Eine Kollision des handgehaltenen zahnärztlichen Geräts mit den anderen Zähnen während der Informationsaufnahme ist hierbei zwangsläufig das Resultat der anatomischen Gegebenheiten. Zur Vermeidung der Kollision wird dann das Gerät üblicherweise leicht schräg gestellt, wodurch sich eine nicht parallele Ausrichtung der Aufnahmevorrichtung gegenüber der molaren Zahnfläche ergibt. Bei der sich hierbei ergebenden, vom Aufnahmewinkel abhängigen perspektivischen Verzeichnung wird allerdings dann die spätere Vergleichbarkeit und/oder Reproduzierbarkeit der diagnostisch aufgenommenen Werte erschwert.

Der vorliegenden Erfindung liegt dementsprechend die Aufgabenstellung zugrunde, eine neuartige Lösung anzugeben, bei der die oben beschriebenen Nachteile vermieden werden.

Die Aufgabe wird durch ein medizinisches Diagnosegerät, welches die Merkmale des Anspruchs 1 aufweist, gelöst. Vorteilhafte Weiterbildungen der Erfindung sind Gegenstand der abhängigen Ansprüche.

Die erfindungsgemäße Lösung beruht auf dem Gedanken, das optische System des Geräts, welches dazu dient, das seitlich einfallende Licht auf die Bilderfassungsmittel zu richten, derart zu modifizieren, dass bei schräg gehaltenem Gerät eine parallele Aufnahme der molaren Zahnfläche erzielt wird bzw. dass der mittlere optische Strahlengang senkrecht auf der Objektebene steht. Hierfür werden ein oder mehrere optische Elemente eingesetzt, welche letztendlich eine Bildumlenkung bewirken, die kleiner als 90° ist. Hierdurch wird eine so genannte orthograde Aufnahmerichtung gegenüber der Längsrichtung des handgehaltenen zahnärztlichen Geräts erreicht, wodurch dessen Handhabung deutlich vereinfacht wird.

Erfindungsgemäß wird dementsprechend ein medizinisches, insbesondere ein zahnmedizinisches Diagnosegerät mit einem länglichen Handstück mit Mitteln zur Bilderfassung sowie einem optischen System mit zumindest einem optischen Element zur Umlenkung eines seitlich von dem Handstück einfallenden Lichtstrahls auf die Mittel zur Bilderfassung vorgeschlagen, wobei erfindungsgemäß der Umlenkwinkel des zumindest einen optischen Elements kleiner als 90° ist.

Bei dem optischen Element zur Umlenkung kann es sich insbesondere um ein im vorderen Endbereich des Handstücks angeordnetes Prisma oder einen Spiegel handeln. Denkbar wäre auch, dass das optische System mehrere Elemente zur Strahlumlenkung aufweist, welche insgesamt eine Umlenkung von weniger als 90° bewirken. Insbesondere hat sich ein Umlenkwinkel von etwa 80° als besonders vorteilhaft herausgestellt.

Ein weiterer Aspekt der vorliegenden Erfindung betrifft Maßnahmen zur Minimierung der Höhe des intraoral eingeführten Teils des Geräts. Hierzu wird eine sehr kurze Brennweite des optischen Systems angestrebt. Eine Vergrößerung des Bildeingangswinkels über den begrenzenden Wert eines im optischen System enthaltenen optischen Elements zur Strahlumlenkung wird dann durch die zusätzliche Verwendung eines strahlaufweitenden optischen Elements erreicht. Dieses strahlaufweitende optische Element befindet sich vorzugsweise räumlich vor dem strahlumlenkenden optischen Element in Richtung Objektebene, also zur molaren Zahnfläche hin.

Gemäß diesem weiteren Aspekt der vorliegenden Erfindung wird dementsprechend ein medizinisches, insbesondere ein zahnmedizinisches Diagnosegerät mit einem länglichen Handstück mit Mitteln zur Bilderfassung sowie einem optischen System mit zumindest einem optischen Element zur Umlenkung eines seitlich von dem Handstück einfallenden Lichtstrahls auf die Mittel zur Bilderfassung vorgeschlagen, wobei erfindungsgemäß das optische System zusätzlich ein bilderweiterndes optisches Element aufweist. Wie zuvor bereits angemerkt, ist dieses bilderweiternde optische Element vorzugsweise räumlich vor dem Element bzw. den Elementen zur Bildumlenkung angeordnet. Es kann sich hierbei insbesondere um eine Linse handeln.

Bei dem erfindungsgemäßen medizinischen Diagnosegerät kann es sich insbesondere um ein Gerät handeln, welches Bestandteil eines Systems zum Transilluminieren von Zähnen ist. Die besondere Ausgestaltung des optischen Systems bringt allerdings auch Vorteile mit sich, wenn lediglich optische Aufnahmen von Zahnoberflächen erstellt werden sollen. Dementsprechend kann die erfindungsgemäße Lösung auch bei klassischen Intraoralkameras zum Einsatz kommen.

Nachfolgend soll die Erfindung anhand der beiliegenden Zeichnung näher erläutert werden. Es zeigen:
- Fig. 1: die Ansicht eines erfindungsgemäßen Ausführungsbeispiels eines zahnärztlichen Diagnosegeräts;
- Fig. 2: den vorderen Endbereich des Geräts von Fig. 1;
- Fig. 3: und 4 zwei Schnittdarstellungen des Diagnosegeräts;
- Fig. 5: eine Schnittdarstellung des vorderen Endbereichs des Geräts und
- Fig. 6: eine schematische Ansicht der erfindungsgemäß ausgestalteten Mittel zur Bildübertragung.
Wie bereits erwähnt wurde stellt das so genannten Transilluminations-Verfahren ein bevorzugtes Anwendungsbeispiel für die vorliegende Erfindung dar. Dessen Funktionsweise soll deshalb nachfolgend kurz grundsätzlich erläutert werden.

Das Transilluminations-Verfahren beruht darauf, einen Zahn mit sichtbarem Licht zu durchleuchten bzw. zu transilluminieren. Hierzu wird mit Hilfe einer Lichtquelle eine Untersuchungsstrahlung generiert, welche auf den Zahn gerichtet wird. Die Untersuchungsstrahlung liegt dabei üblicherweise in einem Wellenlängenbereich von etwa 550 µm bis 790 µm, z. B. bei etwa 670 µm. Das Gewebe des Zahns blockt diese Untersuchungsstrahlung nicht vollständig ab, sondern erlaubt stattdessen, dass das Licht durch den Zahn hindurchtritt. Hierbei wird die Strahlung teilweise gestreut, wobei insbesondere kariöse Bereiche für eine charakteristische Beeinflussung des Lichts sorgen. Wird der auf diese Weise transilluminierte Zahn aus verschiedenen Blickrichtungen betrachtet, so können diese kariösen Bereiche erkannt werden, da diese etwas dunkler erscheinen. Insbesondere dann, wenn Aufnahmen, die zu verschiedenen Zeitpunkten erstellt wurden, miteinander verglichen werden, kann Karies auf diese Weise verhältnismäßig effektiv und auch frühzeitig erkannt werden.

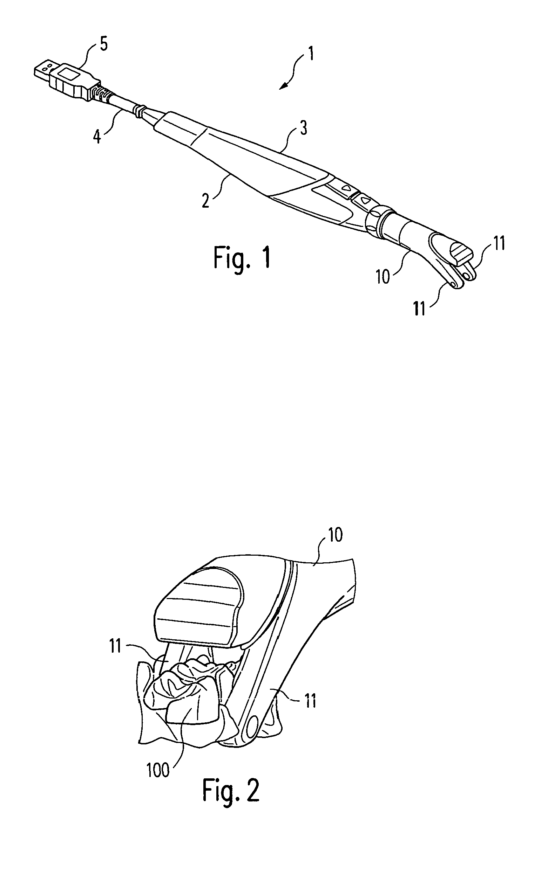

Die auf dem zuvor beschriebenen Verfahren beruhende Untersuchung wird erfindungsgemäß mit Hilfe eines medizinischen Diagnosegeräts durchgeführt, welches in den Fig. 1 und 2 dargestellt ist.

Das allgemein mit dem Bezugszeichen 1 versehene Gerät weist zunächst ein längliches Handstück 2 auf, welches die wesentlichen elektronischen Komponenten sowie die optischen Elemente, die zur Durchführung des Transilluminations-Verfahrens erforderlich sind, beinhaltet. Insbesondere ist innerhalb einer länglichen Griffhülse 3 des Handgeräts 2 eine Bilderfassungseinrichtung bspw. in Form eines CCD-Chips angeordnet, mit deren Hilfe ein optisches Bild des illuminierten Zahns erfasst wird. Ferner findet sich innerhalb der Griffhülse 3 zumindest eine Lichtquelle, deren Licht zum Durchleuchten des Zahns genutzt wird.

Das mit Hilfe der Bilderfassungsmittel erfasste optische Bild des Zahns wird dann einer zentralen Einheit, bspw. einem PC übermittelt, wozu an der Rückseite der Griffhülse 3 ein Kabel 4 mit einem daran befindlichen USB-Stecker 5 vorgesehen ist. Auch andere Verbindungsmöglichkeiten anstelle des USB-Steckers 5 wären denkbar, wobei die Verwendung eines USB-Anschlusses besondere Vorteile mit sich bringt, da dieser dann gleichzeitig auch zur Stromversorgung des Geräts 1 genutzt werden kann.

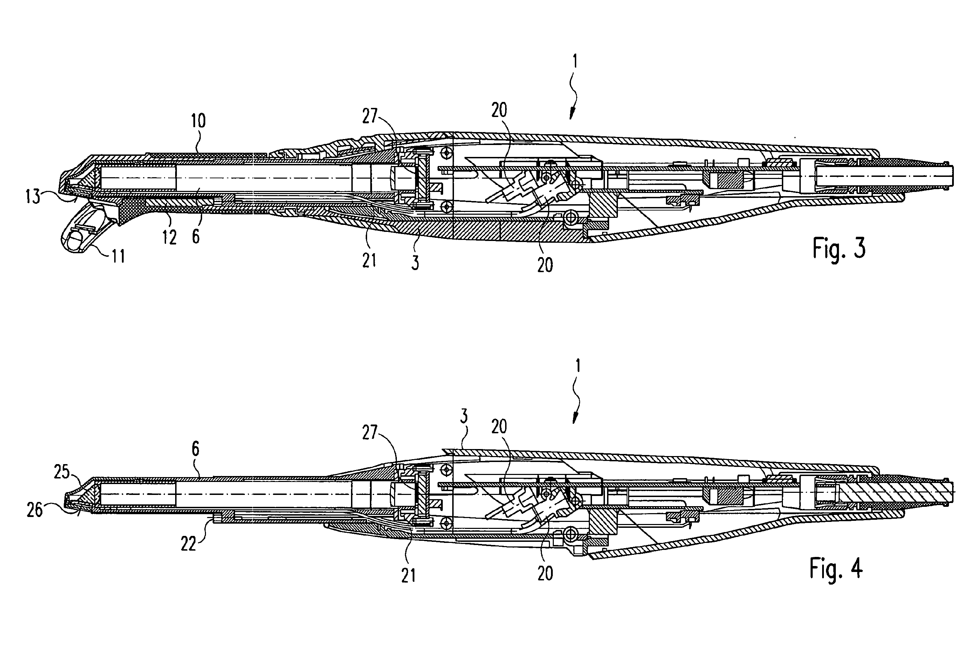

Die Einkopplung des Lichts in den zu untersuchenden Zahn 100 erfolgt mit Hilfe eines Aufsatzes 10, der vorzugsweise lösbar am vorderen Ende der Griffhülse 3 angeordnet ist. Wie den Schnittdarstellungen der Fig. 3 und 4 entnommen werden kann, welche einerseits das Gerät 1 mit dem auf die Griffhülse 3 aufgesetzten Aufsatz 10 (Fig. 3) und andererseits ohne den Aufsatz (Fig. 4) zeigen, weist die Griffhülse 3 an ihrem vorderen Ende einen zylinderförmigen Zapfen 6 auf, auf den der Aufsatz 10 aufgeschoben werden kann. Am vorderen Ende des Aufsatzes 10 befinden sich zwei seitliche Arme 11, welche an den Seitenwänden des zu untersuchenden Zahns 100 bzw. am Zahnfleisch, der so genannten Gingiva, anliegen.

Im zentralen Bereich der Griffhülse 3 sind nunmehr ein oder mehrere Lichtquellen 20 angeordnet, von denen sich aus Lichtleiter 21 bis zu einem vorderen Absatz 22 an dem zylinderförmigen Zapfen 6 erstrecken. Im aufgesetzten Zustand des Aufsatzes 10 sind die vorderen Endbereiche der Lichtleiter 21 auf korrespondierende Lichtleiter 12 ausgerichtet, welche innerhalb des Aufsatzes 10 verlaufen und sich jeweils bis zu den vorderen Endbereichen der Arme 11 erstrecken. Hier befinden sich Auskoppelelemente, welche das Licht von den Lichtleitern seitlich auskoppeln und damit auf die Seitenwände bzw. die Gingiva des Zahns 100 richten, so dass das von den Lichtquellen 20 emittierte Licht effektiv zur Ausleuchtung des Zahns 100 genutzt werden kann.

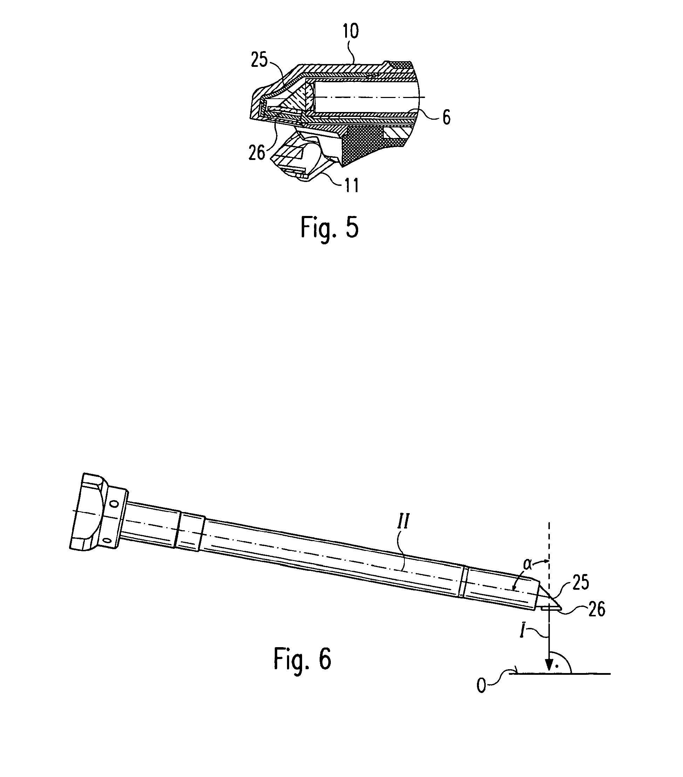

Im vorderen Endbereich des Aufsatzes 10 ist ferner ein Fenster 13 ausgebildet, welches ein Lichteintrittsfenster bildet, über das der zu untersuchende Zahn 100 beobachtet werden kann. Wie insbesondere den Darstellungen der Fig. 3 und 5 entnommen werden kann, erstreckt sich der Zapfen 6 bis in den vorderen Endbereich des Aufsatzes 10, und zwar derart, dass optische Mittel zur Bildumlenkung unmittelbar hinter dem Fenster 13 angeordnet sind. Wie später noch ausführlicher beschrieben wird, wird mit Hilfe dieser optischen Mittel, die durch ein Umlenkprisma 25 und eine davor angeordnete Linse 26 gebildet sind, einfallendes Licht derart umgelenkt, dass es auf einen in der Griffhülse 3 befindlichen CCD-Chip 27 gerichtet wird. Der Chip 27 nimmt dementsprechend ein Bild des sich vor dem Lichteintrittsfenster 13 befindenden Objekts auf.

Wie bereits zuvor beschrieben war es bislang üblich, eine Bildumlenkung um 90° vorzunehmen. Dies führte allerdings zu einer eingeschränkten Bewegungsfreiheit, da das Lichteintrittsfenster 13 des Aufsatzes 10 nicht senkrecht zur Oberfläche eines zu untersuchenden Backenzahns ausgerichtet werden konnte, da dies zu einer Kollision mit den Schneidezähnen führte. Da auf der anderen Seite eine Schrägstellung des Geräts dazu führt, dass keine parallele Ausrichtung der Aufnahmevorrichtung gegenüber der Zahnoberfläche erzielt wird, wird erfindungsgemäß eine spezielle Ausgestaltung der optischen Elemente zur Bildübertragung vorgeschlagen, mit deren Hilfe die zuvor beschriebenen Nachteile vermieden werden und welche nachfolgend näher anhand von Fig. 6 erläutert wird.

Fig. 6 zeigt hierzu in erster Linie die optischen Komponenten zur Bildübertragung, nicht jedoch die weiteren Bestandteile des erfindungsgemäßen Geräts.

Die Besonderheit der erfindungsgemäßen Lösung besteht darin, dass bei schräg gehaltenem Gerät trotz allem eine parallele Aufnahme der molaren Zahnfläche erzielt wird bzw. dass der mittlere Strahlengang I senkrecht auf der Objektoberfläche O steht. Dies wird dadurch erzielt, dass das Umlenkprisma 25 derart ausgestaltet ist, dass es eine Bildumlenkung von weniger als 90° bewirkt. Ein parallel zur optischen Achse I einfallender Lichtstrahl wird also wie in Fig. 6 dargestellt durch das Umlenkprisma 25 um einen Winkel α auf die Achse II umgelenkt, wobei α kleiner als 90°, vorzugsweise etwa 80° ist. Hierdurch ergibt sich eine so genannte orthograde Aufnahmerichtung gegenüber der der Achse II entsprechenden Längsrichtung des Handstücks 2. Mit anderen Worten, wenn das Handstück leicht schräg angesetzt wird, um eine Kollision mit den Schneidezähnen zu vermeiden, wird eine optimale Ausrichtung auf die zu beobachtende Oberfläche O erzielt. Die Qualität der hierdurch erhaltenen Aufnahmen und dadurch die Möglichkeit zur Kariesdiagnose werden deutlich verbessert.

Anzumerken ist, dass anstelle eines einzelnen Umlenkprismas 25 mehrere optische Elemente nacheinander derart angeordnet werden können, dass die Summe des Einflusses der optischen Elemente eine orthograde Aufnahmerichtung gegenüber der Längsachse des handgehaltenen zahnärztlichen Geräts 1 ermöglicht. Mit anderen Worten, die sich durch die Gesamtheit der optischen Elemente ergebende Strahlumlenkung ist wiederum kleiner als 90°. Zur Verbesserung der Reproduzierbarkeit der Aufnahme ist dabei vorzugsweise vorgesehen, dass an dem Aufsatz bzw. an dem Gerät Mittel ausgebildet sind, welche eine Wiederfindung des Winkels der Positionierung des Geräts gegenüber der molaren Zahnfläche ermöglichen. Auf diese Weise ist sichergestellt, dass Aufnahmen im Wesentlichen grundsätzlich aus der gleichen Richtung bzw. Perspektive erfolgen und dementsprechend gut miteinander vergleichbar sind.

Zur Minimierung der Bauhöhe des in den Mundraum des Patienten eingeführten Bereichs des Geräts 1 wird ferner eine sehr kurze Brennweite des optischen Systems angestrebt. Eine Vergrößerung des Bildeingangswinkels wird nunmehr durch die zusätzliche Verwendung eines strahlaufweitenden optischen Elements erzielt, wozu die vor dem Umlenkprisma 25 angeordnete Linse 26 genutzt wird. Mit Hilfe dieser Bildwinkel-erweiternden Maßnahme kann die Qualität der Aufnahmen zusätzlich verbessert werden, wobei diese Maßnahme auch für den Fall einer Bildumlenkung um 90° sinnvoll eingesetzt werden könnte, also auch unabhängig von der zuvor beschriebenen besonderen Ausgestaltung der Strahlumlenkung genutzt werden könnte.

Zur Illumination des zu untersuchenden Zahns 100 wird vorzugsweise eine schmalbandige Beleuchtungsquelle im nahen Infrarotbereich verwendet, die Licht mit einer Wellenlänge vorzugsweise über 600nm, insbesondere im Bereich 780 +/-30nm abgibt. Mit Hilfe der Bilderfassungsmittel wird dann ein Streulichtbild aufgenommen, dessen spektrale Verteilung der Beleuchtungswellenlänge entspricht. Der Bildsensor ist hierzu in einem Bereich von etwa 400nm bis 1000nm empfindlich. Relevant für die Auswertung ist dabei die Wellenlänge im Anregungsbereich, während hingegen der Rest als Rauschen überlagert wird. In das optische System wird vorzugsweise ein Element integriert, welches umgebungsgebundene Störgrößen verringert und damit den Signal-/Rauschabstand vergrößert. Es kann sich hierbei um einen optischen Hochpassfilter zur Verringerung des sichtbaren Lichts unterhalb der Bestrahlungswellenlänge handeln. Alternativ hierzu kann das optische Element auch ein Bandpassfilter sein, welches den Anteil des auf die Bildaufnahmeeinheit erfassten Lichts ober- und unterhalb der Bestrahlungswellenlänge vermindert. Denkbar wäre insbesondere, dieses optische Element durch Modifikation eines bestehenden optischen Elements, z.B. durch die Beschichtung der Linse 26 oder einer anderen Linsenfläche zu realisieren.

Letztendlich wird also durch die erfindungsgemäße Lösung die Qualität der erzielten Aufnahmen des zu untersuchenden Zahns deutlich verbessert, da einerseits die Handhabung des Geräts erleichtert wird und andererseits die optische Erfassung des Zahns optimiert wurde. Die hierbei vorgesehenen Maßnahmen führen grundsätzlich zu Vorteilen, wenn mit Hilfe einer intraoralen Kamera Aufnahmen erstellt werden sollen. Die erfindungsgemäße Lösung ist dementsprechend nicht auf ein System zum Transilluminieren von Zähnen beschränkt sondern kann grundsätzlich auch bei normalen intraoralen Kameras zum Einsatz kommen.

## Patentansprüche

1. Medizinisches, insbesondere zahnmedizinisches Diagnosegerät (1) mit einem länglichen Handstück (2) mit Mitteln zur Bilderfassung (27) sowie einem optischen System mit zumindest einem optischen Element (25) zur Umlenkung eines seitlich von dem Handstück (2) einfallenden Lichtstrahls auf die Mittel zur Bilderfassung (27),
**dadurch gekennzeichnet,**
**dass** der Umlenkwinkel (α) des zumindest einen optischen Elements (25) kleiner als 90° ist.

2. Medizinisches Diagnosegerät nach Anspruch 1,
**dadurch gekennzeichnet,**
**dass** es sich bei dem optischen Element (25) zur Umlenkung um ein im vorderen Endbereich des Handstücks (2) angeordnetes Prisma oder einen Spiegel handelt.

3. Medizinisches Diagnosegerät nach Anspruch 1,
**dadurch gekennzeichnet,**
**dass** das optische System mehrere Elemente zur Strahlumlenkung aufweist, welche insgesamt eine Umlenkung von weniger als 90° bewirken.

4. Medizinisches Diagnosegerät nach einem der vorherigen Ansprüche,
**dadurch gekennzeichnet,**
**dass** der Umlenkwinkel (α) etwa 80° beträgt.

5. Medizinisches Diagnosegerät nach einem der vorherigen Ansprüche,
**dadurch gekennzeichnet,**
**dass** das optische System zusätzlich ein bildwinkelerweiterndes optisches Element (26) aufwei st.

6. Medizinisches, insbesondere zahnmedizinisches Diagnosegerät (1) mit einem länglichen Handstück (2) mit Mitteln zur Bilderfassung (27) sowie einem optischen System mit zumindest einem optischen Element (25) zur Umlenkung eines seitlich von dem Handstück (2) einfallenden Lichtstrahls auf die Mittel zur Bilderfassung (27),
**dadurch gekennzeichnet,**
**dass** das optische System zusätzlich ein bildwinkelerweiterndes optisches Element (26) aufwei st.

7. Medizinisches Diagnosegerät nach Anspruch 5 oder 6,
**dadurch gekennzeichnet,**
**dass** das bildwinkelerweiternde optische Element (26) räumlich vor dem Element bzw. den Elementen zur Bildumlenkung angeordnet ist.

8. Medizinisches Diagnosegerät nach Anspruch 7,
**dadurch gekennzeichnet,**
**dass** das bildwinkelerweiternde optische Element (26) durch eine Linse gebildet ist.

9. Medizinisches Diagnosegerät nach einem der vorherigen Ansprüche,
**dadurch gekennzeichnet,**
**dass** es sich um eine Intraoralkamera handelt.

10. Medizinisches Diagnosegerät nach einem der Ansprüche 1 bis 8,
**dadurch gekennzeichnet,**
**dass** das Gerät Bestandteil eines Systems zum Transilluminieren von Zähnen ist.
